# EUROPÄISCHE PATENTSCHRIFT

(11) **EP 0 928 332 B1**
(45) Veröffentlichungstag und Bekanntmachung des Hinweises auf die Patenterteilung: **03.11.2004**
(21) Anmeldenummer: 97936698.6
(22) Anmeldetag: 09.08.1997
(51) Int. Cl.: C12N 15/00, A01K 67/027

(54) **VERFAHREN ZUR HERSTELLUNG VON SÄUGETIEREN MIT DEFINIERTEN GENETISCHEN EIGENSCHAFTEN**
PROCESS FOR DEVELOPING MAMMALS WITH DEFINITE GENETIC FEATURES
PROCEDE D'OBTENTION DE MAMMIFERES PRESENTANT DES PROPRIETES GENETIQUES DEFINIES

(30) Priorität: 13.08.1996 DE 19632532
(43) Veröffentlichungstag der Anmeldung: 14.07.1999
(73) Patentinhaber: Boehringer Ingelheim International GmbH, 55218 Ingelheim am Rhein (DE)
(72) Erfinder: WAGNER, Erwin, A-1030 Wien (AT); WANG, Zhao-Qi, F-69006 Lyon (FR)
(74) Vertreter: Kläs, Heinz-Gerd, Dr.
(86) Internationale Anmeldenummer: PCT/EP1997/004352
(87) Internationale Veröffentlichungsnummer: WO 1998/006834

(56) Entgegenhaltungen:
- WO-A-97/19589
- NAGY, A. & ROSSANT, J.: "Production of completely ES cell-derived fetuses" 1993 , IN JOYNER, A.L. (ED.) GENE TARGETING: A PRACTICAL APPROACH , IRL PRESS OXFORD XP002049677 in der Anmeldung erwähnt siehe Seite 147 - Seite 180
- UEDA, O. ET AL.: "Production of mice entirely derived from embryonic stem (ES) cell with many passages by coculture of ES cells with cytochalasin B induced tetraploid embryos" EXPERIMENTAL ANIMALS, Bd. 44, Nr. 3, Juli 1995, Seiten 205-210, XP002050187 in der Anmeldung erwähnt
- NAGY, A. ET AL.: "Embryonic stem cells alone are able to support fetal development in the mouse" DEVELOPMENT, Bd. 110, Nr. 3, November 1990, Seiten 815-821, XP002049672 in der Anmeldung erwähnt
- EVERETT C.A. & : "The influence of ploidy on the distribution of cell in chimeric mouse blastocysts" ZYGOTE, Bd. 4, Nr. 1, Februar 1996, Seiten 59-66, XP002049673
- JAMES, R.M. ET AL.: "Restricted distribution of tetraploid cells in mouse tetraploid-diploid chimaeras" DEVELOPMENTAL BIOLOGY, Bd. 167, Nr. 1, 1995, Seiten 213-226, XP002049674 in der Anmeldung erwähnt
- WANG, Z. Q. ET AL.: "Generation of completely embryonic stem cell-derived mutant mice using tetraploid blastocyst injection" MECHANISMS OF DEVELOPMENT, Bd. 62, Nr. 2, 1997, Seiten 137-145, XP002049675
- PRATHER, R.S. ET AL.: "Characterization of DNA synthesis during the 2-cell stage and the production of tetraploid chimeric pig embryos" MOLECULAR REPRODUCTION AND DEVELOPMENT, Bd. 45, Nr. 1, September 1996, Seiten 38-42, XP002049676

## Beschreibung

Die Erfindung bezieht sich auf die Herstellung von nicht-menschlichen Säugetieren mit definierten genetischen Eigenschaften, insbesondere auf die Herstellung transgener Tiere.

Transgene Tiere sind Organismen, in deren Keimbahn permanente genetische Veränderungen eingeführt wurden; ein neu eingeführtes Gen wird als Transgen bezeichnet. Transgene Tiere stellen für die moderne Biologie ein unverzichtbares Werkzeug dar, die gewebsspezifische Regulation von Genen und deren Funktion bei der Entwicklung und bei Erkrankungen zu analysieren. Die transgene Technologie bietet ferner die Möglichkeit, Tiermodelle für Erkrankungen des Menschen zur Verfügung zu stellen sowie große Mengen von Proteinen in Farm-Tieren zu produzieren.

Bei der bisher am häufigsten verwendeten Technik zur Herstellung transgener Tiere wird rekombinante DNA in die befruchteten Eier mikroinjiziert; eine weitere Technik, um Gene in Tierembryonen einzuführen, benützt Viren, meist rekombinante retrovirale Vektoren (siehe den Übersichtsartikel von wagner und Keller, 1992).

Die dritte und jüngste Technik, um fremdes genetisches Material in Tiere einzuführen, macht sich das Potential embryonaler Stammzellen (ES-Zellen) zunutze, chimäre Tiere auszubilden. Säugetierembryonen haben die Fähigkeit, während ihrer Entwicklung fremde Zellen einbauen zu können. Zwei verschiedene Prä-Implantations-Embryonen, für gewöhnlich Morulae, werden *in vitro* aggregiert; dies ergibt einen chimären Embryo, der eine Mischung beider Embryonen darstellt. Diese Embryonen werden anschließend in eine scheinträchtige Maus, die als Ammenmutter fungiert, übertragen; die erhaltenen chimären Nachkommen weisen in ihren Geweben einen unterschiedlichen Anteil von Zellen auf, die jeweils aus einem der beiden ursprünglichen Embryonen stammen. Die Kombination dieser Methode mit der Verwendung von ES-Zellen erwies sich als sehr wirksam für die Herstellung genetisch manipulierter Tiere.

Embryonale Stammzellen leiten sich von der inneren Zellmasse ("inner cell mass", ICM) von Blastozysten ab; sie sind totipotente Zellen, die sich zu sämtlichen Zell-Abstammungsreihen (cell lineages), einschließlich Keimzellen entwickeln können, wenn sie durch Injektion in diploide Blastozysten oder durch Aggregation mit Morulae in einen Embryo eingeführt werden (Robertson, 1987; Bradley, 1987; Beddington and Robertson, 1989; Nagy et al., 1990). ES-Zellen können aus Blastozysten isoliert und anschließend als permanente Zellinien etabliert werden, wenn sie unter gut definierten, genau einzuhaltenden Kulturbedingungen gezüchtet werden; sie können genetisch manipuliert werden. Aufgrund dieser Fähigkeit stellen sie ein wirksames Werkzeug zur Modifikation des Säugetier-, insbesondere des Mausgenoms dar, indem z.B. mit ihrer Hilfe gezielte Mutationen oder andere genetische Veränderungen in die Tiere eingeführt werden (Wagner et al., 1991; Ramirez-Solis et al., 1993; Skarnes, 1993; Bronson and Smithies, 1994).

Seit einiger Zeit stehen Zellen der Bezeichnung "embryonale Keimzellen" ("Embryonic germ cells"; "EG Zellen") zur Verfügung, die aus primordialen Keimzellen zu immortalisierten Zellinien gezüchtet werden können und ES-Zellen in vieler Hinsicht ähnlich sind, u.a. sind EG-Zellen totipotent, können wie ES-Zellen manupuliert werden und bilden Keimbahn-Chimäre aus, wenn sie in Blastozysten eingeführt werden (Donovan et al., 1997).

Im Laufe der letzten Jahre wurden verschiedene experimentelle Techniken entwickelt, um Tiere herzustellen, die von totipotenten Zellen abgeleitet sind. (Totipotente Zellen sind Zellen mit der Fähigkeit, sich zu sämtlichen somatischen Zellen sowie zu Keimzellen zu differenzieren.) Diese Methoden hatten im Fall von ES-Zellen, vorrangig zum Ziel, das gesamte Entwicklungspotential von ES-Zellen in vitro zu erhalten (Williams et al., 1988; Smith et al., 1988) und das Entwicklungspotential der Wirtszellen bei der Bildung von Chimären zu beschränken und somit die Häufigkeit der Bildung von Keimbahn-Chimären zu erhöhen (Nagy et al., 1990; Kaufman und Webb, 1990). Einer der wichtigsten Fortschritte bei der Entwicklung dieser Techniken ist die Verwendung von tetraploiden Embryonen als Wirtszellen, weil tetraploide Zellen nach der Implantation nur ein beschränktes Potential zur Entwicklung aufweisen (Nagy et al., 1990; Kaufmann und webb, 1990; Kubiak und Tarkowski, 1985). Wenn tetraploide mit diploiden Embryonen aggregiert werden, ist die Differenzierung der tetraploiden Zellen weitgehend auf das primitive Endoderm und das Trophectoderm beschränkt, welche in der Folge extraembryonale Gewebe bilden, während die diploiden Zellen den eigentlichen Embryo ausbilden können (James und West, 1994; James et al., 1995).

In einer früheren Arbeit wurden verschiedene ES-Zellinien mit Morulae aggregiert, um auf diese Weise Föten herzustellen, die vollständig von ES-Zellen abstammen (vollständig von ES-Zellen abstammende Organismen werden im folgenden als "ES-Tiere", z.B. "ES-Mäuse", bzw. ES-Föten" bezeichnet); die erhaltenen ES-Föten starben jedoch bei der Geburt (Nagy, 1990). Weitere Studien zeigten, daß lebensfähige und fruchtbare, ausschließlich von ES-Zellen abstammende ES-Mäuse erhalten werden können, wenn Wildtyp-R1-Zellen einer frühen Passage (Nagy et al., 1993) oder TT2-Zellinien (Ueda et al., 1995) für die Aggregation mit tetraploiden Morulae verwendet werden.

ES-Mäuse wurden ferner hergestellt, indem in einem ersten Schritt ES-Zellen in diploide Blastozysten injiziert wurden, womit zunächst chimäre Mäuse erhalten wurden; weitere Kreuzungen ergaben nach zwei Generationen ES-Mäuse. Die Methode der Injektion in Blastozysten wurde zunächst von Gardner, 1968, eine vereinfachte Version von Bradley und Robertson, 1986, sowie von Bradley, 1987, beschrieben.

Das Ziel, durch Verwendung von ES-Zellen aus späteren Passagen lebensfähige ES-Mäuse zu erhalten, wurde mit den bisher verfügbaren Methoden nicht erreicht (Nagy et al., 1993); die Herstellung von ES-Mäusen unter Verwendung von genetisch modifizierten ES-Zellen schien überhaupt nicht möglich. (Die Verwendbarkeit von ES-Zellen aus späteren Passagen ist vor allem im Hinblick auf die Verwendung von Zellinien von Bedeutung, ferner im Hinblick auf Verwendung genetisch veränderter ES-Zellen, deren Selektion naturgemäß mit einer Erhöhung der Passagenanzahl einhergeht.)

Der vorliegenden Erfindung lag die Aufgabe zugrunde, ein neues Verfahren bereitzustellen, mit dem nicht-menschliche Säugetiere mit definierten genetischen Eigenschaften, insbesondere transgene Säugetiere, erhalten werden können, die vollständig von ES-Zellen Zellen abgeleitet sind.

Die gestellte Aufgabe wird durch ein Verfahren zur Herstellung von nicht-menschlichen Säugetieren mit definierten genetischen Eigenschaften, insbesondere transgenen Säugetieren, gelöst, bei dem embryonale Stammzellen derselben Säugetierspezies in Blastozysten eingeführt werden und der erhaltene Embryo in eine Ammenmutter transferiert wird. Das verfahren ist dadurch gekennzeichnet, daß embryonale Stammzellen mit definierten genetischen Eigenschaften in tetraploide Blastozysten eingeführt werden.

Mit Hilfe des erfindungsgemäßen Verfahrens können Tiere erhalten werden, die vollständig von ES-Zellen abgeleitet sind. Das erfindungsgemäße Verfahren bietet den Vorteil, in einem einzigen Schritt aus in vitro gezüchteten ES-Zellen vollständig von ES-Zellen abgeleitete Tiere zu erhalten.

Unter den Begriff "transgene Säugetiere" fallen im Rahmen der vorliegenden Erfindung definitionsgemäß Tiere, die eine permanente genetische Veränderung beliebiger Art tragen.

Tiere, die "vollständig von ES-Zellen abgeleitet" sind, enthalten vorzugsweise zu 100% Zellen, die von ES-Zellen stammen. Die Tiere können jedoch einen geringen Anteil, vorzugsweise nicht mehr als 10%, an Zellen aufweisen, die von den tetraploiden Blastozysten abgeleitet sind.

In einer bevorzugten Ausführungsform der Erfindung sind die Säugetiere Mäuse; das Verfahren kann jedoch grundsätzlich auf sämtliche Säugetiere Anwendung finden, von denen ES-Zellen erhalten werden können. Voraussetzung dafür, ES-Zellen von anderen Säugetieren als Mäusen zu erhalten, ist die Definition von Bedingungen, die die Züchtung von ES-Zellen aus diesen Organismen und die Etablierung von ES-Zellinien erlauben, u.a. das Erfordernis von spezifischen Wachstumsfaktoren sowie von Feeder-Zellen für die Co-Kultivierung mit den ES-Zellen. Diese Bedingungen können mit Hilfe von Serienversuchen empirisch ermittelt werden.

Die Isolierung von ES-Zellen aus Blastozysten, die Etablierung von ES-Zellinien und deren anschließende Züchtung werden nach herkömmlichen Methoden vorgenommen, wie z.B. von Doetchmann et al., 1985; Li et al., 1992; Robertson, 1987; Bradley, 1987; Wurst und Joyner, 1993; Hogan et al., 1994; Wang et al., 1992, beschrieben. ES-Zellinien, z.B. Maus-ES-Zellinien, können in Vorversuchen darauf getestet werden, ob sie aufgrund ihres Entwicklungspotentials für die Verwendung im Rahmen der vorliegenden Erfindung geeignet sind. Dazu können Zellen der interessierenden Linien in diploide Maus-Embryonen injiziert, die erhaltenen Embryonen in Ammenmütter eingebracht und die Jungen auf Chimärismusrate sowie auf Häufigkeit der Ausbildung von Keimbahnchimären untersucht werden.

Tetraploide Blastozysten können nach bekannten Methoden durch Elektrofusion von Zwei-Zell-Embryonen erhalten und anschließend gezüchtet werden, wie z.B. von James et al., 1992; Nagy und Rossant, 1993; oder von Kubiak und Tarkowski, 1985 beschrieben.

Die Einführung der ES-Zellen in die Blastozysten wird ebenfalls in an sich bekannter Weise vorgenommen. Bevorzugt im Rahmen der vorliegenden Erfindung ist die Methode der Mikroinjektion, wie z.B. von Wang et al., 1991, beschrieben. Bei der herkömmlichen Mikroinjektion werden in einer Mikromanipulationsapparatur ca. 5-10 ES-Zellen, die einer Einzel-Zellsuspension entnommen wurden, in eine Blastozyste, die mittels Haltepipette immobilisiert wurde, injiziert. Dann werden die Embyonen mindestens 3 h lang, gegebenenfalls über Nacht, inkubiert.

In einer bevorzugten Ausführungsform der Erfindung werden genetisch manipulierte ES-Zellen verwendet, um transgene Tiere zu erhalten.

Bezüglich der Art der genetischen Veränderung der ES-Zellen gibt es keinerlei Beschränkungen: Es können Gene überexprimiert, mutiert oder, im Hinblick auf die Herstellung von sog. "Knock-Out"-Tieren, ausgeschaltet werden; ferner können Fremdgene inseriert oder intrachromosomale Deletionen vorgenommen werden. Die genetische Veränderung kann auf einem oder auf beiden Allelen vorgenommen werden, letzterer Ansatz wurde z.B. von Hilberg und Wagner, 1992, für die Ausschaltung des c-jun-Gens beschrieben. Daß die vorliegende Erfindung eine genetische Veränderung auf beiden Allelen ermögllicht, ist ein besonderer Vorteil; mit den Methoden des Standes der Technik war es nur möglich, nach weiteren Kreuzungen und langwieriger Züchtung von Tieren, die eine genetische Veränderung auf einem Allel aufwiesen, transgene Tiere zu erhalten, in denen beide Allele die gewünschte Veränderung trugen.

Für die genetische Manipulation der ES-Zellen können herkömmliche Methoden verwendet werden. Im allgemeinen werden Plasmide, vorzugsweise linearisierte Plasmide, verwendet, die die gewünschte genetische Veränderung tragen. Im Hinblick auf die Selektionierbarkeit der genetisch veränderten ES-Zellen enthalten die Plasmide zweckmäßig ein Marker-Gen, z.B. das Neomycin-, Hygromycin- oder Puromycinresistenzgen, unter Kontrolle eines Promotors. Im Hinblick auf die Expression eines auf dem Plasmid enthaltenen Gens in den Wirtszellen kann das Plasmid Genexpressionskontrollsequenzen, z.B. einen in ES-Zellen funktionellen, starken Promotor, wie den PGK (Phosphoglycerinkinase)-Promotor, enthalten.

Die Methoden, mit denen das Plasmid in die Zellen eingeführt wird, sind literaturbekannte Standardmethoden für den *in vitro* Transfer von DNA in Säugetierzellen, z.B. Elektroporation, Calciumphosphat-Präzipitation, oder Methoden auf der Grundlage der Rezeptor-vermittelten Endozytose, geoffenbart z.B. in der WO 93/07283.

Eine weitere Methode zur Einführung genetischer Veränderungen in den ES-Zellen benutzt Viren, z.B. rekombinante retrovirale Vektoren; bzgl. auf dem Vektor enthaltenen Sequenzabschnitten, die die Selektion genetisch veränderter Zellen bzw. die Expression in der Zelle ermöglichen, gilt grundsätzlich das zu den Plasmiden Gesagte (Wagner und Keller, 1992; Stewart et al., 1985).

Mit Hilfe des erfindungsgemäßen Verfahrens ist es möglich, lebensfähige und fruchtbare transgene Säugetiere, insbesondere ES-Mäuse, aus *in vitro* genetisch veränderten ES-Zellen routinemäßig herzustellen.

Mit Hilfe des erfindungsgemäßen Verfahrens können u.a. aus genetisch manipulierten ES-Zellen, die z.B. ein spezifisches Gen überexprimieren oder in denen ein spezifisches Gen inaktiviert wurde, transgene Tiere reproduzierbar hergestellt werden, die u.a. für Genfunktionsstudien oder für die Produktion von Proteinen verwendet werden können. Das erfindungsgemäße Verfahren bietet gegenüber herkömmlichen Methoden zur Herstellung transgener Tiere eine leistungsfähige, schnelle und wirtschaftliche Möglichkeit, mutante Tierföten, insbesondere Mausföten, sowie transgene Stämme direkt aus totipotenten Zellen, in denen die gewünschten genetischen Veränderungen vorgenommen wurden, herzustellen.

Alle drei im Rahmen der vorliegenden Erfindung untersuchten ES-Zellinien der Bezeichnung D3, R1 und GS1 bildeten nach Injektion in diploide Blastozysten Keimbahn-Chimären aus. Bei Injektion in tetraploide Blastozysten wurden lebende ES-Mäuse aus R1- und GS1-Zellen erhalten. Mit D3-Zellen gelang es nicht, auch nicht mit Zellen einer frühen Passage (Passage 9), nach Injektion von ES-Zellen in tetraploide Blastozysten lebende ES-Mäuse herzustellen. Dies steht im Einklang mit früheren Beobachtungen aus Aggregationsversuchen (Nagy et al, 1990; siehe auch Tab.2) mit diesen Zellen. Die Unfähigkeit von D3-Zellen, lebensfähige ES-Mäuse zu bilden, ist vermutlich nicht darauf zurückzuführen, daß diese Zellen ihr Entwicklungspotential verloren haben; D3-Zellen wurden bereits häufig in sog. "Gene-Targeting"- Versuchen eingesetzt, wobei nach deren Injektion in diploide Blastozysten eine hohe Chimärismusrate und Keimbahn-Chimären erhalten werden konnten (Urbánek et al., 1994; Wang et al., 1992; Wang et al., 1994; siehe auch Tab.1). Es ist jedoch möglich, daß das Potential von D3-Zellen, zu einigen Zelltypen zu differenzieren, die kritisch für die Anpassung des Fötus an das postnatale Leben sind, aufgrund unbekannter genetischer oder epigenetischer Veränderungen beeinträchtigt ist. Diese Annahme wird durch die Beobachtung gestützt, daß D3-Zellen in der Lage sind Föten zu produzieren, die sich zum normalen Geburtstermin entwickeln, daß jedoch die Neugeborenen nicht in der Lage sind, die Atmung aufrechtzuerhalten, sowie daß sie ein erhöhtes Körpergewicht und Polytaktylie aufweisen und bei der Geburt sterben. Diese Eigenschaften erinnern an die phänotypischen Merkmale von Mäusen, denen das "imprinted" Igf2/Mpr-Gen fehlt (Wang et al., 1994; Lau et al., 1994); es könnte daher sein, daß geprägte ("imprinted") Gene oder Gene, welche das Wachstum der Föten regulieren, für den beobachteten Effekt verantwortlich sind. Während in der Umgebung von Wirtszellen diploider Embryonen fehlerhafte Funktionen der ES-Zellen von den Wirtszellen komplementiert werden dürften, scheint das den D3-Zellen aufgrund ihrer Undifferenziertheit eigene Entwicklungspotential, alle funktionellen Zelltypen bilden zu können, in einer vollständig von ES-Zellen stammenden Umgebung eingeschränkt zu sein. Die Einführung von verschiedenen Wildtyp-ES-Zellen in tetraploide Embryonen, zweckmäßig in Serienversuchen, kann daher als schneller und sicherer Test dienen, um die Eignung von ES-Zellen für die Verwendung im Rahmen der vorliegenden Erfindung zu überprüfen.

Der genetische Hintergrund der jeweiligen Mausstämme, von denen die verschiedenen ES-Zellen stammen, dürfte ein weiterer Faktor sein, der die Lebensfähigkeit der ES-Mäuse beeinflußt. Alle im Rahmen der vorliegenden Erfindung verwendeten ES-Zellinien stammten vom Mausstamm 129 ab: die R1-Zellen stammten von einer Maus-Blastozyste aus einer Kreuzung zwischen den Substämmen 129/Sv und 129/Sv-CP (Nagy et al., 1993) ab; GS1-Zellen stammten von 129/Sv/Ev. D3-Zellen (Doetchmann et al., 1985) und J1-Zellen (Li et al., 1992) stammten aus 129/Sv bzw. 129/terSv. TT2-Zellen, die ebenfalls ES-Mäuse ergaben, stammten von einem hybriden Stamm F1 (C57BL/6 x CBA) (Yagi et al., 1993). Aufgrund der im Rahmen der vorliegenden Erfindung erhaltenen Ergebnisse sowie früheren Arbeiten (Nagy et al., 1993,Ueda et al., 1995) kann nicht ausgeschlossen werden, daß ES-Zellinien, die von verschiedenen Mausstämmen oder Substämmen abgeleitet sind, unterschiedliche Fähigkeiten zur Bildung lebensfähiger ES-Mäuse aufweisen.

Die Effizienz bei der Herstellung neugeborener ES-Mäuse mittels Injektion von Wildtyp-R1-Zellen in tetraploide Embryonen (14%) war höher als die Herstellung mittels Aggregation (6% im Rahmen der vorliegenden Erfindung bzw. 7% in der von Nagy et al. (1993) beschriebenen Arbeit). Dieses Ergebnis ist in Übereinstimmung mit einem Vergleich zwischen der Methode der Aggregation und der Injektion von ES-Zellen in diploide Embryonen (Wood et al., 1993). Die erfindungsgemäße Verwendung von tetraploiden Blastozysten für die Injektionsmethode ergab, daß einige ausgewählte R1-Zellklone, die länger als 24 Passagen in vitro kultiviert worden waren (z.B. R169.2.3 und R-*fra*3), noch die Fähigkeit aufwiesen, lebensfähige ES-Mäuse zu produzieren. Diese Ergebnisse sind bemerkenswert, insbesondere angesichts von Resultaten früherer Aggregations-Versuche, in denen Wildtyp-R1-Zellen ihre Fähigkeit, lebensfähige ES-Mäuse zu produzieren, nach Passage 14 verloren (Nagy et al., 1993). Die Gründe, warum die Injektion von ES-Zellen in tetraploide Blastozysten reproduzierbar zur Ausbildung von ES-Mäusen führt, sind nicht vollständig geklärt. Da ES-Zellen ursprünglich aus ICM von Blastozysten gewonnen werden und diesen ICM-Zellen auch sehr ähnlich sind (Beddington und Robertson, 1989), ist es vorstellbar, daß sowohl die räumliche Nähe von ES-Zellen und ICM als auch deren Kompatibilität in der Entwicklung für diesen Effekt verantwortlich sind. Diese Annahme wird darüberhinaus durch die Beobachtung aus Vergleichsversuchen gestützt, daß die Effizienz, chimäre Mäuse zu erzeugen, geringer war, wenn ES-Zellen in diploide Morulae unterhalb der "zona pellucida" eingebracht wurden, als wenn die herkömmliche Blastozysten-Injektionsmethode (Injektion in diploide Blastozysten) verwendet wurde.

Die hohe Effizienz der erfindungsgemäßen Methode macht sie gegenüber den Methoden des Standes der Technik (Aggregation von ES-Zellen mit tetraploiden Blastozysten bzw. Injektion in diploide Blastozysten) überlegen und bietet die zur Zeit einzige Möglichkeit, mutante Mäuse direkt von genetisch veränderten totipotenten Zellen zu erzeugen.

Die Erzeugung von lebensfähigen mutanten Mäusen direkt von genetisch manipulierten ES-Zellen hat viele Vorteile. Da die fötalen Gewebe vollständig von totipotenten Zellen abstammen, die genetisch modifizierbar sind, bietet diese Technik einen direkten Weg, um fötales Material reinen ES-Zell-Ursprungs für zellbiologische, molekularbiologische oder genetische Studien herzustellen (Forrester et al., 1991; Carmeliet et al., 1996).

ES-Föten reproduzieren die Expressionsmuster von spezifischen Genen, wie z.B. des *Pax*5-Gens bzw. einem "trapped" Gen, im Vergleich mit Föten, die durch Kreuzungen von heterozygoten mutanten Mäusen, die aus denselben ES-Zellen hervorgegangen sind, verläßlich. Vorteilhafterweise können ES-Föten für Expressionsstudien verwendet werden, weil dadurch eine rasche Produktion von fötalem Material (einige Tage) ermöglicht wird, während die konventionelle Züchtung normalerweise vier bis fünf Monate dauert. Darüberhinaus minimiert das verläßliche und reproduzierbare Expressionsmuster in ES-Föten mögliche Komplikationen in herkömmlichen chimären Geweben, die definitionsgemäß sowohl aus Wildtyp- als auch aus mutierten ES-Zellen bestehen. Daher ist diese Technik für Studien über Genfunktion oder zur Identifikation von neuen Genen, z:B: in "gene-trap" Studien (Skarnes, 1993) nützlich. Es wurde gezeigt, daß mit Hilfe der erfindungsgemäßen Methode (Injektion von ES-Zellen in tetraploide Blastozysten), mutante Mauslinien, z.B. c-*fos*-Transgene und *fra*-1-"knock-out"-Mäuse, direkt von mutanten ES-Zellen in effizienter Art und Weise hergestellt werden können. Das erfindungsgemäße Verfahren bietet die Möglichkeit, aus ES-Zellen, die für die Integration und Expression von Transgenen vorselektiert wurden, transgene Mauslinien zu produzieren. Die Effizienz, Mäuse, die ein spezifisches Gen überexprimieren, herzustellen, wird dadurch - im Vergleich mit der herkömmlichen Injektionsmethode, bei der diploide Blastozysten verwendet werden - signifikant verbessert. Das erfindungsgemäße Verfahren wurde in mehreren Gen-Überexpressions- und Inaktivierungsstudien angewandt.

Darüberhinaus ist es möglich, mit dieser Methode mutante Gewebe zum Studium spezifischer Effekte herzustellen, falls Inaktivierung oder Überexpression zum Tod oder zu beeinträchtigter Gametogenese in heterozygoten Mutanten oder gar in Chimären führen sollte (z.B. siehe Carmeliet et al., 1996).

Letztlich bietet das erfindungsgemäße Verfahren die Möglichkeit, mutante Tierstämme, insbesondere Mausstämme, rasch und wirtschaftlich herzustellen und einen schnellen Zugriff auf mutante Föten und Tiere zu haben, was einen wesentlichen Vorteil für die Forschung auf dem Gebiet der Mausgenetik darstellt.

Außer für die Herstellung transgener Tiere kann das erfindungsgemäße Verfahren für die Herstellung von nicht genetisch veränderten ES-Tieren, die bestimmte erwünschte Eigenschaften aufweisen, verwendet werden. Dazu werden ES-Zellen eingesetzt, die im Hinblick auf die erwünschten Eigenschaften durch Züchtungsversuche vorselektiert werden, um identische Tiere mit den entsprechenden Eigenschaften zu erhalten.

### Figurenübersicht

- Fig.1:: Herstellung von tetraploiden Embryonen. Injektion von ES-Zellen in tetraploide Blastozysten
- Fig.2:: GPI-Analyse von neugeborenen ES-Mäusen und von Nachkommen von ES-Mäusen, die von R1-Zellen abstammen
- Fig.3:: Vergleich der *lacZ*-Genexpression in ES-Föten und in Föten, die von heterozygoten Kreuzungen stammen

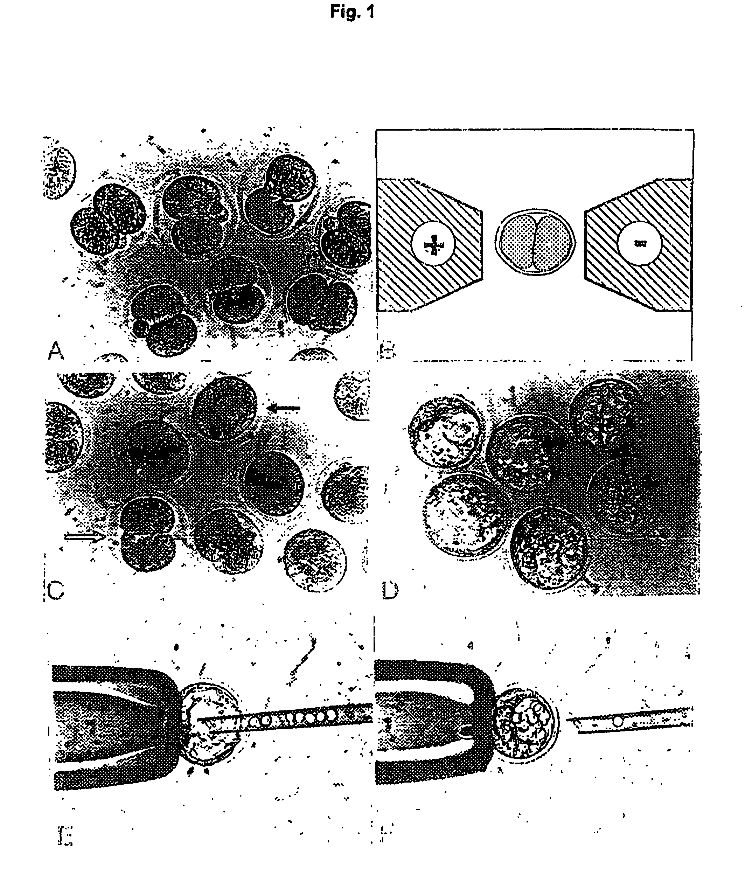

In den folgenden Beispielen, die die vorliegende Erfindung veranschaulichen, wurden, wenn nicht anders angegeben, die folgenden Materialien und Methoden verwendet:
a) Mäuse: Als Spender diploider Embryonen wurden C57BL/6-Mäuse und als Spender tetraploider Embryonen B6CBAF1-Mäuse (C57BL/6 x CBA) verwendet. Beide Stämme sind homozygot für das Gpi-1^{b}-Allel am *Gpi*-1 Locus, das für die Glukosephosphatisomerase (GPI) kodiert.
b) ES-Zellen und Gentransfer: Es wurden folgende in der Literatur beschriebene ES-Zellen verwendet:
   D3-Zellen (Doetchmann et al., 1985)
   R1-Zellen (Nagy et al., 1993)
   J1-Zellen (Li et al., 1992)
   GS1-Zellen wurden aus Blastozysten des Substammes 129/Sv/EV isoliert. Dieser Mausstamm wurde aus einer chimären Maus, erhalten nach Keimbahnübertragung von ES-Zellen des Genotyps von AB1, etabliert. AB1-ES-Zellen waren ursprünglich vom Substamm 129/Sv/EV etabliert worden, wie von McMahon und Bradley, 1990, und von Papaioannou, 1993, beschrieben. Zur Isolierung der GS1-Zellen wurde im wesentlichen die von Robertson, 1987, beschriebene Methode verwendet: Blastozysten wurden auf einer 4-Well-Platte auf Feeder-Zellen plattiert, die ICM vermehrte sich nach 5 Tagen Kultur. Die ES-Zell-ähnlichen Klumpen wurden mit einer Pipette zerteilt und auf eine neue Platte mit Feederzellen neu ausplattiert. Die expandierten ES-Zellen wurden identifiziert und weiter auf ihren Karyotyp und ihre Totipotenz hinsichtlich der Entwicklung untersucht.
   Alle ES-Zellen wurden ursprünglich aus der Vermehrung von Blastozysten isoliert, die vom Mausstamm 129, der für das Gpi-1^{a}-Allel homozygot ist, erhalten wurden. Um die ES-Zellen zu modifizieren, wurden R1-Zellen der Passage 16 mit verschiedenen Konstrukten elektroporiert; G418-resistente Klone wurden ausgewählt und vor der Injektion expandiert. Es wurden die folgenden Konstrukte verwendet: ein c-fos überexprimierender Vektor (Wang et al., 1991); ein sog. "Gene-Trap-Vektor" der Bezeichnung pSAβgeo, der ein *lacZ-neo* Fusionsgen ohne Promotor enthält und die Eigenschaft hat, in Introns beliebiger Gene integrieren zu können (Friedrich und Soriano, 1991); ein sog. "Gene-Targeting-Vektor", der das *Pax*5-Gen durch homologe Rekombination in Mäusen unterbricht (Urbánek et al., 1994); sowie ein Gene-Targeting-Vektor, der durch homologe Rekombination das endogene *fra*-1 Gen ("Fos related antigen 1") unterbricht. (Zur Herstellung des *fra*-1 Gene-Targeting-Vektors wurden aus einer genomischen Mausbibliothek einige Cosmid-Klone isoliert, die das *fra*-1 Gen enthalten, und es wurden die Sequenz und die Exon/Intron-Organisation des gesamten Gens bestimmt. Aufgrund dieser Information wurde durch homologe Rekombination in ES-Zellen eine Null-Mutation im *fra*-1-Gen eingeführt. Ausgehend vom Plasmid pGNA (Le Mouellic et al., 1990; 1992) wurde ein Gene-Targeting-Vektor konstruiert. In diesem waren die essentiellen DNA-Bindungs- und Dimerisierungs(Leucinzipper)-Domänen von *fra*-1 durch die bakteriellen Gene, kodierend für β-Galaktosidase und Neomycinresistenz, welche in Säugetieren als Reportergen bzw. selektierbarer Marker dienen, ersetzt. Nach der Elektroporation von ES-Zellen wurden G418-resistente Kolonien entweder mittels Southern Blot oder Färbung auf β-Galaktosidase-Aktivität analysiert, um die Integration der Vektoren zu bestätigen.
c) Gewinnung von Zwei-Zell-Embryonen; Elektrofusion: Zwei-Zell-Embryonen wurden aus weiblichen B6CBAF1-Mäusen am Tag 1.5 post-coitum (p.c.) isoliert und für die Herstellung von tetraploiden Blastozysten verwendet (Fig. 1A). Mittels Elektrofusion wurde Tetraploidie der Embryonen herbeigeführt, wobei das von Nagy und Rossant (1993) beschriebene Verfahren modifiziert wurde: Zwei-Zell-Embryonen wurden in einer 0.3 M Mannitollösung 30 sec lang äquilibriert, bevor sie einzeln zwischen zwei Platinelektroden in 0.3M Mannitol angeordnet und einem kurzen Stromstoß bei 95V während 30 psec in einem effektiven Feld von 2V unter Verwendung eines Stromstoßgenerators CF-100 (Biochemical Laboratory Service, Budapest) ausgesetzt wurden (Fig.1B). Nach einer Inkubationszeit von 15 min begannen zwei Blastomere zu fusionieren (Fig.1C, offener Pfeil) und allmählich einen Ein-Zell-Embryo zu bilden (Fig.1C, Pfeil).
d) Aggregation von ES-Zellen mit Morulae; Injektion von ES-Zellen in Blastozysten: Für die Aggregation wurden die Morulae entweder aus den Eileitern von trächtigen Mäusen (Tag 2.5 p.c.) isoliert oder aus tetraploiden Ein-Zell-Embryonen erhalten, indem diese 24 bis 40 h nach der Elektrofusion kultiviert wurden. Die Aufarbeitung und Aggregation der ES-Zellen wurde durchgeführt, wie von Nagy et al. (1990) beschrieben. Diploide Blastozysten wurden aus dem Uterus von trächtigen C57BL/6-Mäusen (Tag 3.5 p.c.) isoliert. Um tetraploide Blastozysten zu erhalten, wurden die elektrofusionierten Ein-Zell-Embryonen 48 bis 60 h nach der Fusion kultiviert (M16 Medium bei 37°C in einem Inkubator mit 95% Luft/5% CO₂; Fig.1D). Wildtyp- oder genmanipulierte ES-Zellen wurden dann nach der von Wang et al. (1991) beschriebenen Methode in diploide bzw. tetrapoide Blastozysten injiziert (Fig.1E, Fig.1F). Während sich die injizierten diploiden Embryonen in der normalen Tragzeit entwickelten und dann natürlich entbunden wurden, wurden die trächtigen Mäuse, die tetraploide Embryonen empfangen hatten, am Tag 18.5 einem Kaiserschnitt unterzogen (Nagy et al., 1990). Lebensfähige Föten, beurteilt aufgrund von Herzschlag und Atmung, wurden von einem Weibchen aufgezogen, das am selben Tag oder am Vortag geworfen hatte. Einige der lebenden Jungen wurden auf GPI-Marker untersucht. Die überlebenden ES-Mäuse wurden weiter mit Wildtyp-C57BL/6-Mäusen gepaart, um sie auf Fruchtbarkeit und Keimbahnvererbung zu testen.
e) GPI-Isoenzym-Analyse: Verschiedene Gewebe von Föten oder erwachsenen Tieren, die von ES-Zellen abstammten, wurden isoliert und in destilliertem Wasser zerkleinert. Die Proben wurden durch drei Gefrier-/Auftauzyklen lysiert und, wie von Bradley (1987) und Wang et al. (1991) beschrieben, nach Proteinextraktion der GPI-Analyse unterworfen. In den chimären Geweben wurde der Anteil der Zellen, die von ES-Zellen oder vom Wirt stammten, aus dem Verhältnis der GPI-1A- bzw. GPI-1B- Isoenzymaktivität, die in einem gekoppelten optischen Assay sichtbar gemacht wurde, geschätzt.
f) β-Galaktosidase-Histochemie: Es wurde die von Sanes et al. (1996) beschriebene Methode in modifizierter Form verwendet, um die β-Galaktosidase-Aktivität in fixierten intakten Embryonen zu bestimmen. Die Embryonen und ihre extra-embryonalen Membranen wurden 5-10 min lang fixiert (100 mM Na-Phosphat, pH 7.4, 5 mM EGTA, 2 mM MgCl₂, 0.2% Glutaraldehyd), anschließend dreimal gewaschen (100 mM Na-Phosphat, pH 7.4, 2 mM MgCl₂ 0.01% Na-Desoxycholat, 0.02%. NP-40) und mit einer histochemischen Reaktionsmischung (100 mM Na-Phosphat, pH 7.4, 2 mM MgCl₂ 0.01% Na-Desoxycholat, 0.02% NP-40, 5mM K-Eisen(III)-Zyanid, 5mM K-Eisen(II)-Zyanid und 1mg/ml X-Gal) gefärbt.

### Beispiel 1

### Herstellung chimärer Mäuse, die von diploiden Embryonen und Wildtyp-ES-Zellen stammen

Um das Entwicklungspotential verschiedener ES-Zellinien zu testen, wurden zunächst vier verschiedene Wildtyp-ES-Zellinien verwendet, um chimäre Mäuse herzustellen, und zwar einerseits durch Aggregation der ES-Zellen mit diploiden Morulae, andererseits durch deren Injektion in diploide Blastozysten. Von allen vier ES-Zellinien konnte gezeigt werden, daß sie in der Lage sind, nachdem sie in diploide Maus-Embryonen eingebracht wurden, eine hohe Chimärismusrate zu liefern sowie Keimbahnchimären mit großer Häufigkeit zu bilden (siehe Tab.1). Interessanterweise wurde ein hoher Anteil von weiblichen Chimären auch mit R1- und J1-Zellen erhalten, von denen einige die Fellfarbe Agouti des Stammes 129/Sv an ihre Nachkommenschaft vererbten (Tab.1).

### Beispiel 2

### Herstellung lebensfähiger ES-Mäuse durch Aggregation von ES-Zellen mit tetraploiden Embryonen oder Injektion der Zellen in Blastozysten

Um tetraploide Embryonen herzustellen, erhielten Zwei-Zell-Embryonen einen kurzen Stromstoß, der zur Fusion von ca. 90% der Embryonen führte. Diese Embryonen wurden weiterkultiviert. Es wurden fünf Versuche durchgeführt, in denen sich die Embryonen mit hoher Frequenz zu Morulae (68-95%) und zu Blastozysten (80-98%) entwickelten. Die Morulae wurden für die Aggregation mit ES-Zellen verwendet, die Blastozysten, um in sie ES-Zellen zu injizieren (Fig.1E, Fig.1F). Es wurden alle vier Wildtyp-ES-Zellinien (D3, R1, J1 und GS1) im Hinblick auf die Herstellung von ES-Mäusen getestet. Aus der Aggregation mit D3-Zellen wurden nach Kaiserschnitt 26 lebende Neugeborene erhalten, von denen jedoch kein einziges überlebte (Tab.2). Ebenso konnten aus J1-Zellen keine lebensfähigen ES-Mäuse erhalten werden (Tab.2). Im Gegensatz dazu ergaben R1-Zellen nach Aggregation mit tetraploiden Morulae ES-Mäuse mit ähnlicher Häufigkeit (siehe Tab.2) wie von Nagy et al., 1993 beschrieben.

Da die Methode der Injektion von ES-Zellen in diploide Blastozysten hinsichtlich Chimärenbildung ähnlilch effizient war wie die Aggregationsmethode (siehe auch Wood et al., 1993), wurde als nächstes untersucht, ob ES-Mäuse mittels Injektion von Wildtyp-ES-Zellen in tetraploide Blastozysten erhalten werden können. Bei Verwendung von D3-Zellen aus Passage 9 konnte ein relativ hoher Anteil (28%) von vollentwickelten Föten am Tag 18.5 p.c. (E18.5) mittels Kaiserschnitt erhalten werden (Tab.2); die Neugeborenen konnten jedoch die Atmung nicht aufrecht erhalten und starben kurz darauf. Interessanterweise wiesen diese Neugeborenen ein höheres Körpergewicht sowie Polydaktylidie auf. Von 36 tetraploiden Blastozysten, die mit R1-Zellen (Passage 14) injiziert worden waren, wurden neun lebensfähige Junge mittels Kaiserschnitt zum Zeitpunkt E18.5 geboren (Tab.2). Fünf davon konnten die Atmung aufrecht erhalten und wurden von einer Ammenmutter aufgezogen. Bedauerlicherweise waren zwei Junge nach 7 Tagen nicht auffindbar und eines starb im Alter von 5 Wochen. Zwei dieser ES-Mäuse überlebten bis zum Erwachsenenalter und zeigten vollständige Keimbahnvererbung (Tab.2). Nach Injektion von GS1-Zellen in 54 tetraploide Blastozysten entwickelten sich 17 Embryonen in der normalen Tragzeit (Tab.2). Sechs Junge wurden mittels Kaiserschnitt geboren und konnten ihre Atmung aufrecht erhalten. Fünf Junge starben innerhalb von 48 h, und eine ES-Maus überlebte bis zum Erwachsenenalter und vererbte das aus ES-Zellen stammende genetische Material an die Nachkommenschaft (Tab.2).

### Beispiel 3

### Herstellung von ES-Mäusen mit genetisch modifizierten ES-Zellklonen

Nachdem aus tetraploiden Blastozysten, in die R1- oder GS1- Wildtyp-Zellen injiziert worden waren, lebensfähige ES-Mäuse entstanden waren, wurde als nächstes untersucht, ob die erfindungsgemäße Methode auch geeignet ist, mutante ES-Mäuse aus genetisch manipulierten ES-Zellen zu liefern. Als erstes wurden R1-Zellen mit dem c-fos-Expressionsvektor elektroporiert und zwei G418-resistente Klone der Bezeichnung R-169.2.3 und R-169.2.5 für die Injektion in tetraploide Blastozysten verwendet (die R1-Klone wurden vor ihrer Injektion in Blastozysten über mehr als 24 Passagen kultiviert). Klon R-169.2.5 wurde in insgesamt 103 Blastozysten injiziert; es wurden zwölf Neugeborene mittels Kaiserschnitt erhalten. Drei von ihnen hielten die Atmung aufrecht, starben aber nach 48 h. Klon R-169.2.3 ergab eine höhere Zahl von überlebenden Neugeborenen; 23 Junge waren nach dem Kaiserschnitt lebensfähig, zwölf davon wurden von einer Ammenmutter aufgezogen (Tab.2). Leider starben sieben Neugeborene in den ersten drei Tagen aufgrund ungenügender Pflege durch die Ammenmutter. Zwei weitere Mäuse gingen während der Entwöhnungsphase verloren. Drei Mäuse überlebten bis zum Erwachsenenalter. Nachdem mittels Southern Blot Analyse bestätigt worden war, daß das Transgen auf die Nachkommen vererbt wurde, wurden zwei transgene Linien etabliert.

In einem weiteren Versuch wurde ein R1-Klon der Bezeichnung R-*fra* 3 (*fra*-1 +/-) aus Passage 24 verwendet, in dem ein Allel des fra-1-Gens durch homologe Rekombination unterbrochen ist. R-*fra* 3-Zellen wurden in 48 tetraploide Blastozysten injiziert; mittels Kaiserschnitt wurden acht lebende Junge erhalten. Vier von fünf von einer Ammenmutter aufgezogene Neugeborenen erreichten das Erwachsenenalter, von drei davon konnte bestätigt werden, daß sie das mutierte Allel (*fra*-1 +/-) an die Nachkommen vererbten (Tab.2). Die weibliche chimäre Maus war steril, was im Gegensatz zu den mit Wildtyp-R1-Zellen erhaltenen Ergebnissen stand, wo mit diesen Zellen und diploiden Embryonen Chimären-Weibchen erhalten wurden, die Keimbahn-Nachkommen hervorbringen konnten.

### Beispiel 4

### GPI-Analyse in Geweben von ES-Föten und ES-Mäusen

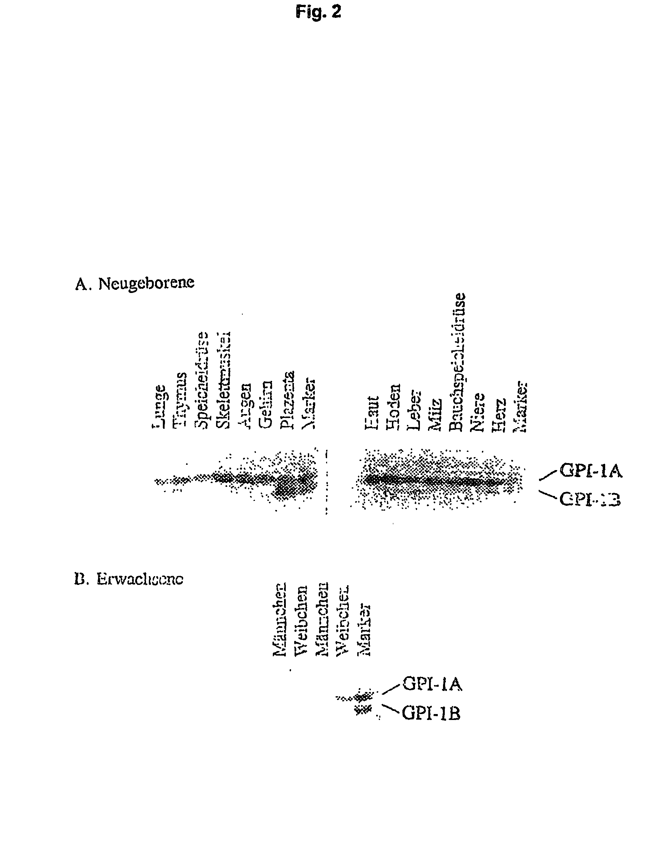

Um zu bestätigen, daß die nach den obigen Beispielen erhaltenen Föten und erwachsenen Mäuse tatsächlich ausschließlich von ES-Zellen abstammen, wurde eine GPI-Analyse durchgeführt, mit der der Beitrag der ES-Zellen zur Gewebsbildung bestimmt werden kann. Aus den Versuchen, in denen eine Aggregation vorgenommen worden war, zeigten alle elf Föten aus D3-Zellen, einer aus R1-Zellen und einer aus J1-Zellen in allen untersuchten Geweben eine 100%ige Abstammung von ES-Zellen (Tab.3). Ein von R1-Zellen abgeleiteter Fötus wies einen kleinen Gewebsanteil (ca. 10%) im Herzen auf, der von Wirtszellen abstammte, wobei die anderen untersuchten Gewebe ausschließlich von ES-Zellen abstammten (Fig.2A, Tab.3). Gleichermaßen stammten die meisten ES-Föten und alle R1- und GS1-abgeleiteten erwachsenen ES-Mäuse, die mittels Injektion der Zellen in tetraploide Blastozysten hergestellt worden waren, ausschließlich von ES-Zellen ab, mit der Ausnahme von zwei der D3-abgeleiteten Föten (E18.5), die in Herz, Lunge und Leber einen Beitrag von Wirtszellen im Ausmaß von 10 bis 50% aufwiesen. Bemerkenswerterweise zeigten vier von R1-Zellen abstammende Föten bereits im frühen Stadium (Tag 13.5 p.c.) ausschließlich den ES-spezifische GPI-1A Marker (Tab.3). Zusätzlich wurden die Nachkommen der von R1 abstammenden ES-Mäuse mittels GPI-Analyse untersucht, wobei gezeigt werden konnte, daß sie von ES-Zellen abstammen.

ES-Föten und erwachsene Mäuse, die durch Injektion von genetisch modifizierten R1-Zellen in tetraploide Blastozysten hergestellt worden waren, wurden ebenfalls mittels GPI-Analyse untersucht. Von Geweben von zwei neugeborenen Jungen (E18.5), die vom ES-Klon R-169.2.5 abgeleitet waren, zeigte sich, daß sie vollständig von ES-Zellen abstammten. Die GPI-Analyse von drei Tage alten Jungen (abgeleitet von D3-Zellen) und erwachsenen Mäusen, die mit R1-Zellen hergestellt worden waren, die entweder ein c-fos-Transgen überexprimierten (R-169.2.3) oder ein inaktiviertes Allel von *fra*-1 (R*-fra* 3) aufwiesen, zeigte in allen untersuchten Geweben, daß diese zu 100% von ES-Zellen abstammten (Tab.3). Des weiteren wurden an den Nachkommen dieser ES-Mäuse GPIund Southern Blot-Analysen durchgeführt. Dabei wurde bestätigt, daß nur der GPI-1A-Marker in allen Nachkommen vorhanden war; einige erbten auch entweder das Transgen (*c-fos*) oder das unterbrochene Allel *fra*-1. Fig.2A zeigt die GPI-Analyse von neugeborenen ES-Mäusen, die von R1-Zellen stammen. Alle Gewebe, außer Plazenta und Herz, enthielten nur den GPI-1A-Marker, was auf eine 100%ige Abstammung von ES-Zellen hinweist. Fig.2B zeigt die GPI-Analyse der Nachkommenschaft einer ES-Maus: Das Blut von vier Jungen einer erwachsenen ES-Maus enthielt ausschließlich das GPI-1A-Isoenzym, was die ES-Abstammung der Nachkommenschaft bestätigt.

### Beispiel 5

### Vergleich der Genexpressionsmuster von ES-Zell-Embryonen und Keimbahn-Embryonen

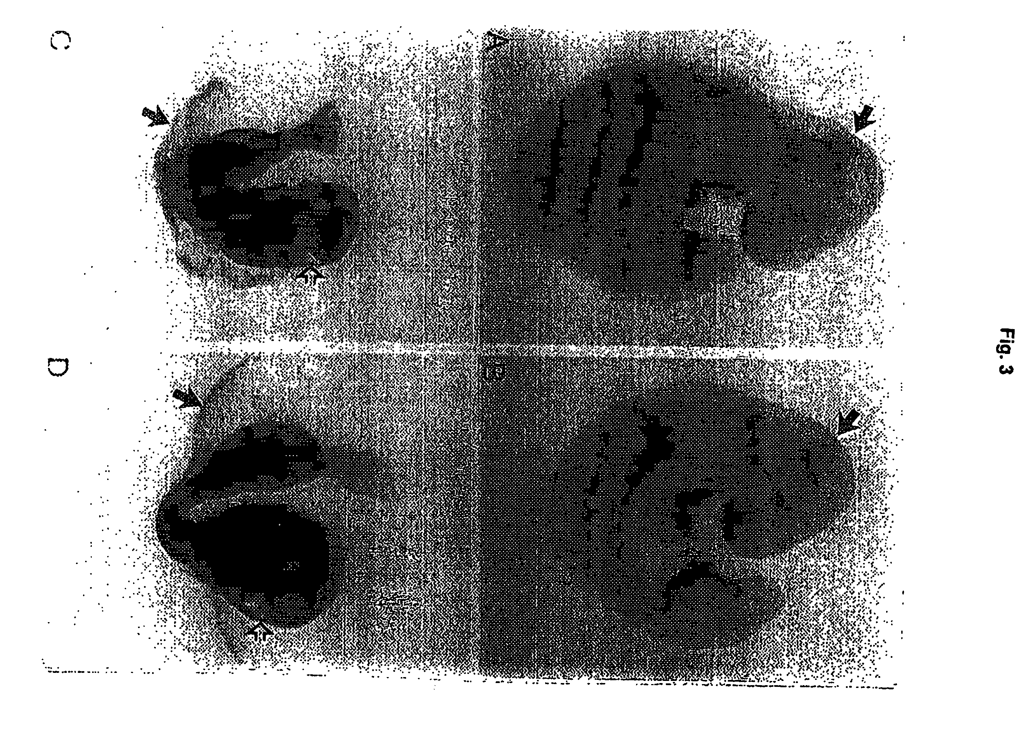

Um die Eignung des erfindungsgemäßen Verfahrens zur Herstellung von ES-Mäusen für die Untersuchung von Genexpression und mutanten Phänotypen zu prüfen, wurde untersucht, wann und wo spezifische Gene in ES-Zell-Embryonen und in Keimbahn-Embryonen exprimiert wurden. Für diese Untersuchung wurden zwei genetisch manipulierte ES-Zellklone, die das lacZ-Reportergen enthielten, ausgewählt, wobei einer zu einem sehr beschränkten *lacZ*-Expressionsmuster führte (*Pax*5 +/- ES-Klon D3-15; Urbánek et al., 1994); während der andere eine ausgedehnte β-Galaktosidase-Färbung ergab (vgl. unten). Fig.3 zeigt den Vergleich der *lacZ*-Genexpression in ES-Föten (Fig.3A,C) mit der in Föten, die aus heterozygoten Kreuzungen (Fig.3B,D) stammten. E9.5-Embryonen, erhalten von tetraploiden Blastozysten, die mit dem Klon D3-15 injiziert worden waren, zeigten eine spezifische Expression des lacZ-Gens an der Grenze zwischen Mittel- und Hinterhirn (Fig.3A, Pfeil). Dieses Färbemuster war identisch mit dem von Embryonen, die aus heterozygoten Kreuzungen erhalten wurden (Fig.3B; siehe auch Urbánek et al., 1994). Der zweite untersuchte ES-Klon war ein R1-Klon der Bezeichnung R-βgeo3, der aus einem Gene-Trap-Experiment erhalten wurde. R-βgeo3-Zellen wurden in diploide und tetraploide Blastozysten injiziert. Die injizierten diploiden Embryonen ergaben fruchtbare Chimären, einige von ihnen vererbten das *lacZ*-Transgen an ihre Nachkommen. Es dauerte ca. vier bis fünf Monate, eine solche transgene Mauslinie zu etablieren und Embryonen aus heterozygoten Kreuzungen verfügbar zu haben. Embryonen, erhalten mittels Injektion von R-βgeo3-Zellen in tetraploide Blastozysten, wurden am Tag 8.5 isoliert und auf β-Galaktosidase-Aktivität gefärbt. Im gesamten eigentlichen Embryo, in Amnionmembran (Fig.3C, offener Pfeil) und Allantois wurde eine intensive Färbung nachgewiesen, nicht jedoch im Dottersack (Fig.3C, Pfeil). Dieses Färbemuster war identisch mit dem Färbemuster in heterozygoten Embryonen, die nach heterozygoter Kreuzung erhalten worden waren (Fig.3D). Diese Ergebnisse zeigen, daß das Expressionsmuster des Transgens in ES-Mäusen über Keimbahnvererbung zuverlässig erhalten bleibt.

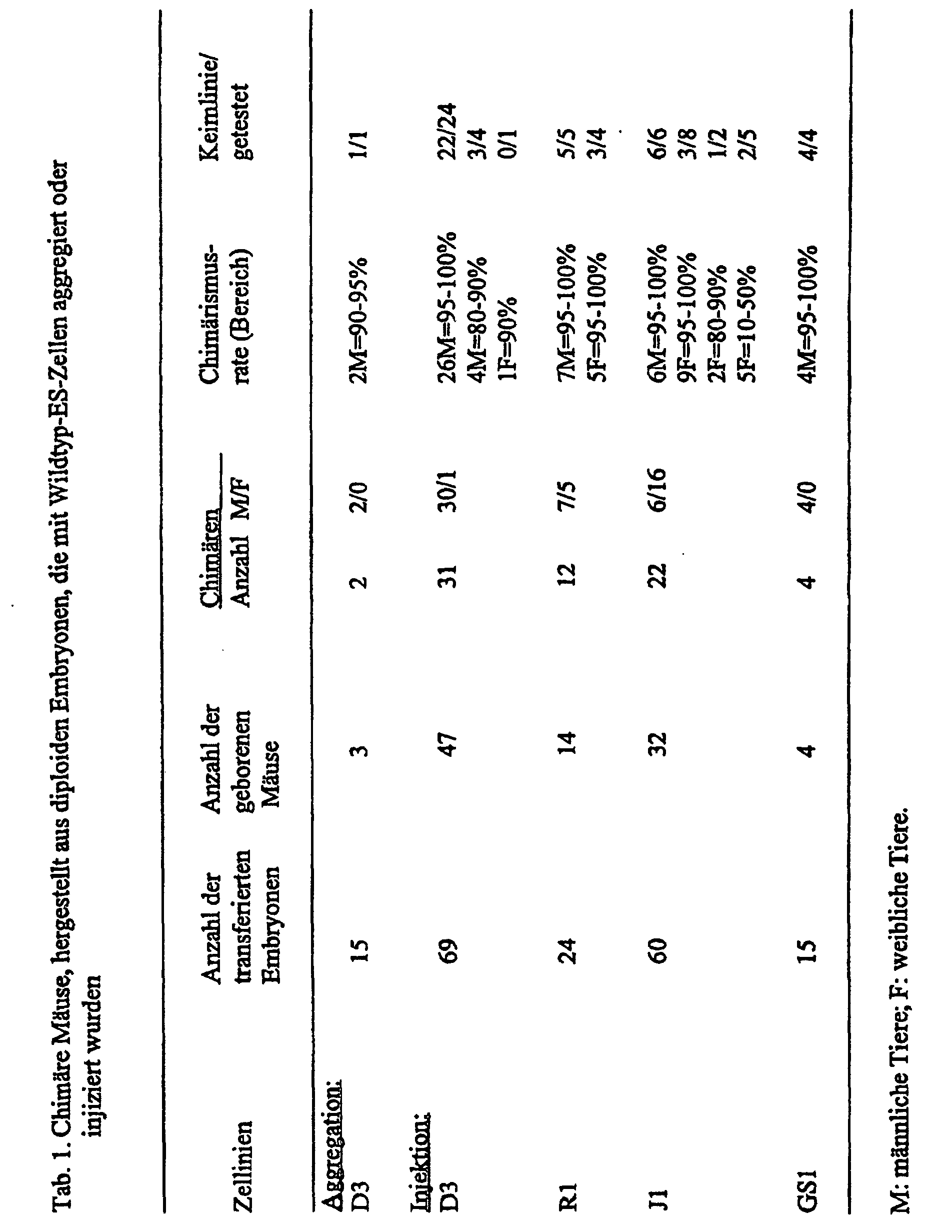

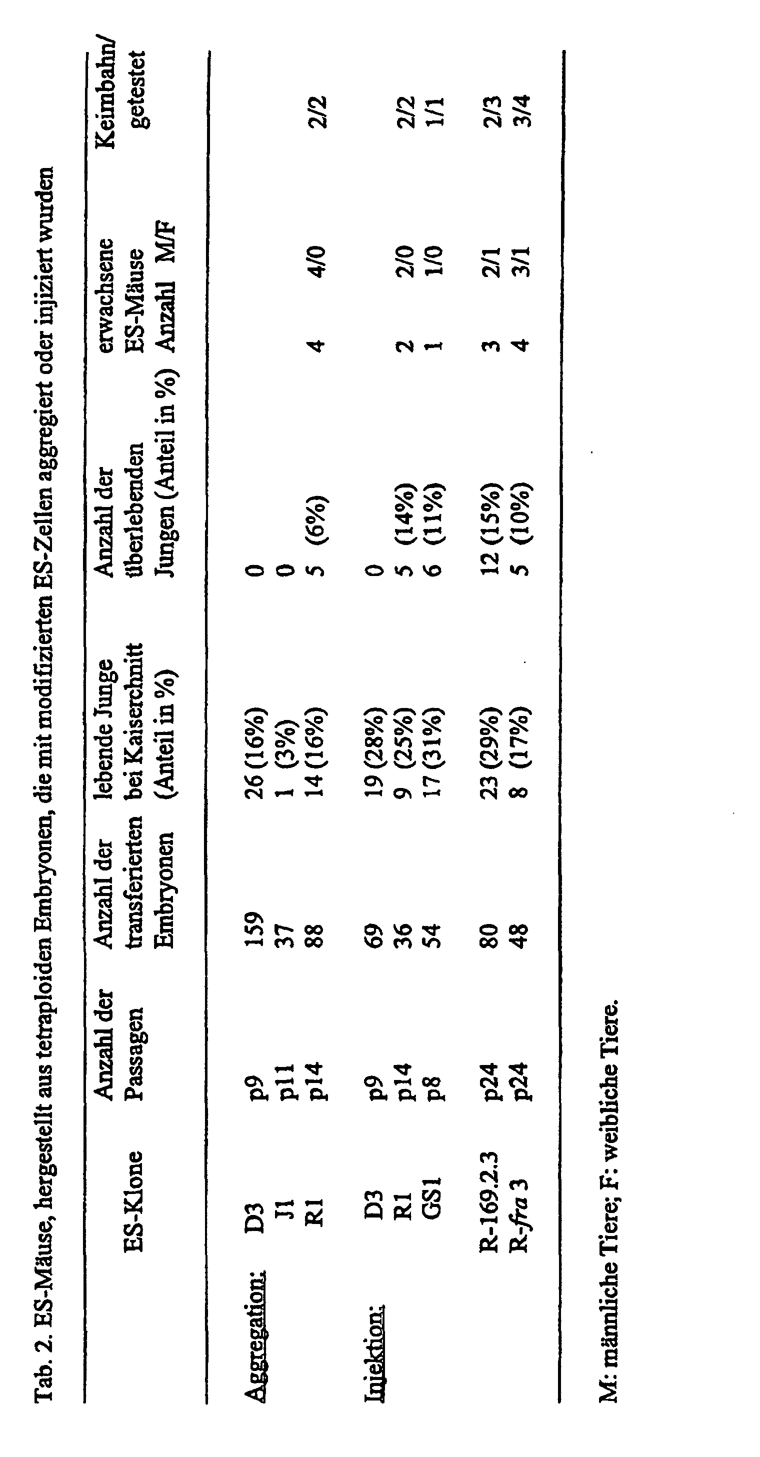

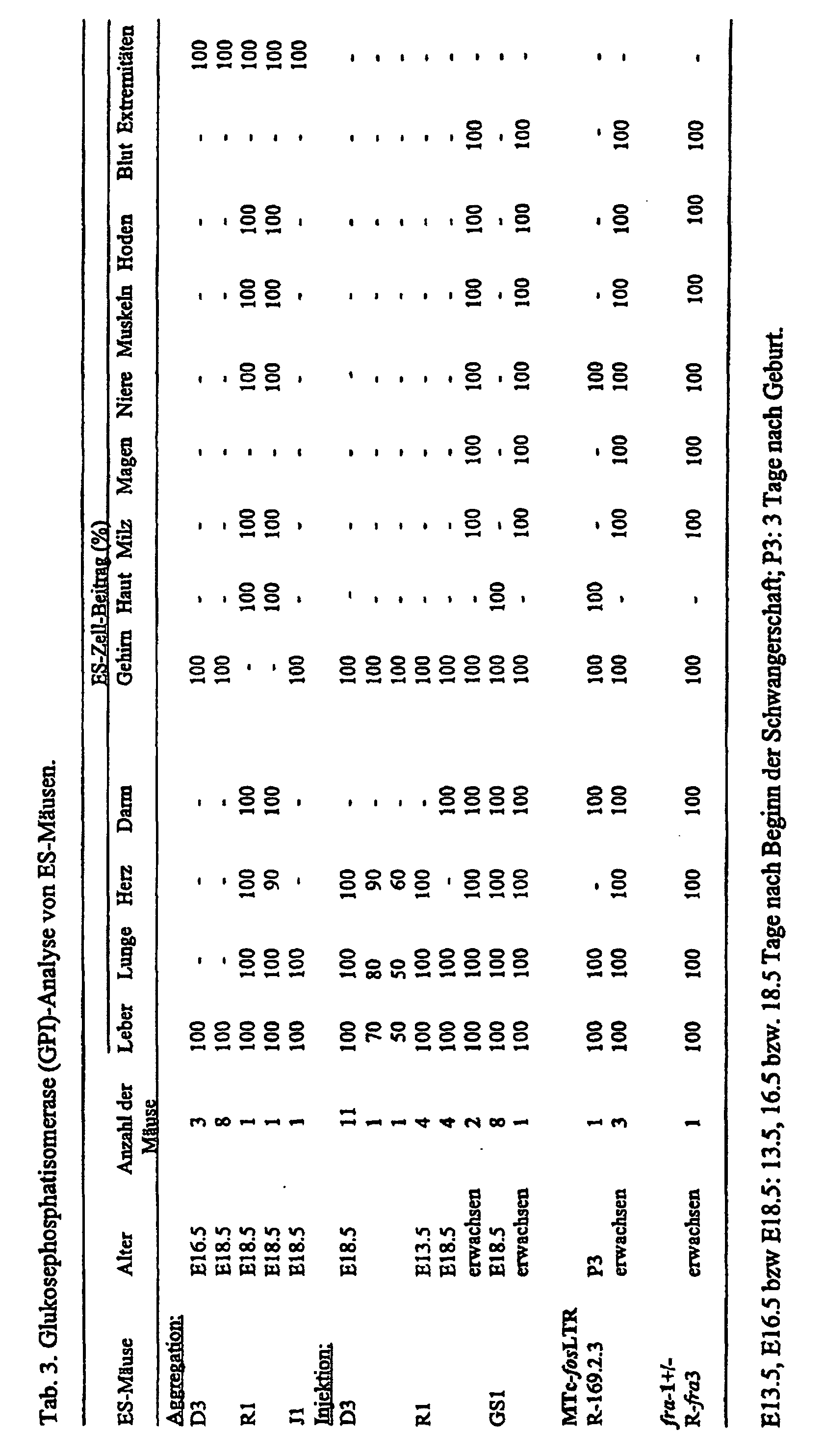

### LITERATUR

Beddington, R. S. P., & Robertson, E. J. (1989) *Development* 105, 733-737.

Bradley, A. (1987) in Teratocarcinomas and *Embryonic Stem* Cells: *A practical approach*, ed. Robertson, E. J. (IRL Press, Oxford Washington DC), 113-151.

Bradley, A. und Robertson, E. J., 1968, in *Manipulating* the mouse *embryo,* A Laboratory Manual. eds. Hogan, R., Constantini, F. und Lacy, E. (Cold Spring Harbor Laboratory Press), 188-195

Bronson, S. K., & Smithies, O. (1994) J. *Biol.* Chem. 269, 27155-27158.

Carmeliet, P., Ferreira, V., Breier, G., Pollefeyt, S., Keickens, L., Gertsenstein, M., Fahrig, M., Vandenhoeck, A., Harpal, K., Eberhardt, C., Declercq, C., Pawling, J., Moons, L., Collen, D., Risau, W., & Nagy, A. (1996) Nature 380, 435-439.

Chambers, C. A., Kang, J., Pawling, J., Huber, B., Hozumi, N., & Nagy, A. (1994) *Proc. Natl.* Acad. *Sci. USA.* 91, 1138-1142.

Doetchmann, T. C., Eistetter, H., Katz, M., Schmidt W., & Kemler, R. (1985) J. Embryol. *Exp.* Morph. 87, 27-45.

Donovan, P.J., et al., 1997, Transgenic Animals, Generation and Use, Editor: L.M:Houdebine, pp 179-187, Harwood Academic Publishers.

Forrester, L. M., Bernstein, A., Rossant, J., & Nagy, A. (1991) *Proc. Natl. Acad. Sci. USA* **88,** 7514-7517.

Friedrich, G., & Soriano, P. (1991) *Genes & Dev.* **5,** 1513-1523

Gardner, 1968, Nature 220, 596-597

Hilberg, F. & Wagner, E.F. (1992) *Oncogene* **7,** 2371-2380 Hogan, B., Beddington, R., Costantini, F. & Lacy, E. (1994) in *Manipulating* the *Mouse Embryo: A* laboratory manual, eds. Hogan, B., Beddington, R., Costantini, F. & Lacy, E. (Cold Spring Harbor Laboratory Press) pp. 253-290)

James, R. M., & West, J. D. (1994) *Hum.* Genet. **93,** 603-604.

James, R. M., Kaufman, M. H., Webb, S., & West, J. D. (1992) Genet. *Res. Camb.* **60,** 185-194.

James, R. M., Klerkx, A. H. E. M., Keighren, M., Flockhart, J. H., & West J. D. (1995) *Dev. Biol.* **167,** 213-226.

Kaufman, M. H., & Webb, S. (1990) *Development* **110,** 1121-1132.

Kubiak, J. Z., & Tarkowski, A. K. (1985) *Exp. Cell Res.* **157,** 561-566.

Lau, M. M. H., Stewart, C. E. H., Liu, Z., Bhartt, H., Rotwein, P., & Stewart, C. L. (1994) *Genes & Dev.* **8,** 2953-2963.

Le Mouellic, H., Lallemand, Y. und Brulet, P. (1990) *Proc. Natl. Acad. Sci.* USA **87**, 4712-4716.

Le Mouellic, H., Lallemand, Y. und Brulet, P. (1992) *Cell* **69,** 251-264.

Li, E., Bestor, T. H., & Jaenisch, R. (1992) Cell **69,** 915-926.

McMahon, A. P. und Bradley, A., 1990, *Cell* **62,** 1073-1085

Nagy, A., & Rossant, J. (1993) in *Gene Targeting: A practical approach,* ed, Joyner,A. L. (IRL Press, Oxford New York Tokyo), pp. 147-180.

Nagy, A., G6cza, E., Diaz, E. M., Prideaux, V. R., Ivänyi, E., Markkula, M., & Rossant, J. (1990) *Development* **110**, 815-821.

Nagy, A., Rossant, J., Nagy, R., Abramow-Newerly, W., & Roder, J. (1993) *Proc. Natl. Acad. Sci. USA* **90**, 8424-8428.

Papaioannou, V. und Johnson, R., 1993, in Gene *Targeting: A practical approach,* ed. Joyner, A. L. (IRL Press, Oxford, New York, Tokyo) 147-180

Ramirez-Solis, R., Davis A. C., & Bradley, A. (1993) *Methods Enzymol.* **225**, 855-878.

Robertson, E. J. (1987) in *Teratocarcinomas and Embryonic Stem Cells: A practical approach,* ed. Robertson, E. J. (IRL Press, Oxford Washington DC), pp. 71-112.

Sanes J. R., Rubenstein J. L. R., & Nicolas, J.-F., 1986, *EMBO J.* **5**, 3133-3142.

Skarnes, W. C. (1993) *Current Opinion* in *Biotech.* **4,** 684-689.

Smith, A. G., Heath, J. K., Donaldson, D. D., Wong, G. G., Moreau, J., Stahl, M., & Rodgers, D. (1988) *Nature* **336,** 688-690.

Stewart, C.L., Vanek, M. & Wagner, E.F. (1985) *EMBO* Journal **4,** 3701-3709.

Ueda, O., Jishage, K., Kamada, N., Uchida, S., & Suzuki, H. (1995) Jikken-Dobutsu **44,** 205-210.

Urbánek, P., Wang, Z.-Q., Fetka, I., Wagner, E. F., & Busslinger, M. (1994) *Cell* **79,** 901-912.

Wagner, E. F., Wang, Z.-Q., Grigoriadis, A. E., Möhle-Steinlein, U., Aguzzi, A., & Risau, W. (1991) in *Origins of Human Cancer: A Comprehensi ve Review,* (Cold Spring Harbor Laboratory Press), 815-823.

Wagner, E.F. & Keller, G. (1992) in *Development, The Molecular Genetic* Approach, (Springer, Heidelberg), pp. 440-458.

Wang, Z.-Q., Fung, M. R., Barlow, D. P., & Wagner, E. F. (1994) Nature 372, 464-467.

Wang, Z.-Q., Grigoriadis, A. E., Möhle-Steinlein, U., & Wagner, E. F. (1991) *EMBO J.* **10,** 2437-2450.

Wang, Z.-Q., Ovitt, C., Grigoriadis, A. E., Möhle-Steinlein, U., Rüther, U. & Wagner, E. F. (1992) *Nature* **360,** 741-744.

Williams, R. L., Hilton, D. J., Pease, S., Willson, T. A., Stewart, C. L., Gearing, D. P., Wagner, E. F., Metcalf, D., Nicola, N. A., & Gough, N. M. (1988) *Nature* **336**, 684-687.

Wood, S. A., Allen, N. D., Rossant, J., Auerbach, A., & Nagy, A. (1993) *Nature* **365**, 87-89.

Wurst, W. & Joyner, A. L. (1993) in *Gene Targeting: A practical approach,* ed. Joyner, A. L. (IRL Press, Oxford New York Tokyo), pp. 147-180.

Yagi, T., Tokunaga, T., Furuta, Y., Nada, S., Yoshida, M., Tsukada, T., Saga, Y., Takeda, N., Ikawa, Y., & Aizawa, S. (1993) Anal. *Biochem.* **214,** 70-76.

## Patentansprüche

1. Verfahren zur Herstellung von nicht-menschlichen Säugetieren, die von embryonalen Stammzellen abgeleitet sind, mit definierten genetischen Eigenschaften, insbesondere transgenen Säugetieren, bei dem embryonale Stammzellen derselben Säugetierspezies in Blastozysten eingeführt werden und der erhaltene Embryo in eine Ammenmutter transferiert wird, **dadurch gekennzeichnet, daß** embryonale Stammzellen mit definierten genetischen Eigenschaften in tetraploide Blastozysten eingeführt werden.

2. Verfahren nach Anspruch 1, **dadurch gekennzeichnet, daß** die Säugetiere Mäuse sind.

3. Verfahren nach Anspruch 1 oder 2, **dadurch gekennzeichnet, daß** Blastozysten verwendet werden, die mittels Elektrofusion von Zwei-Zell-Embryonen und anschließender Züchtung erhalten wurden.

4. Verfahren nach einem der vorhergehenden Ansprüche, **dadurch gekennzeichnet, daß** die embryonalen Stammzellen mittels Mikroinjektion in tetraploide Blastozysten eingeführt werden.

5. Verfahren nach einem der Ansprüche 1 bis 4, **dadurch gekennzeichnet, daß** genetisch manipulierte embryonale Stammzellen verwendet werden.

6. Verfahren nach Anspruch 5, **dadurch gekennzeichnet, daß** die genetisch manipulierten embryonalen Stammzellen durch Einführung von Plasmiden, die die gewünschte genetische Veränderung tragen, erhalten wurden.

## Claims

1. Process for producing non-human mammals derived from embryonic stem cells, having defined genetic properties, particularly transgenic mammals, wherein embryonic stem cells of the same mammalian species are introduced into blastocysts and the resulting embryo is transferred into a foster mother, **characterised in that** embryonic stem cells with defined genetic properties are introduced into tetraploid blastocysts.

2. Process according to claim 1, **characterised in that** the mammals are mice.

3. Process according to claim 1 or 2, **characterised in that** blastocysts are used which have been obtained by electrofusion of two-cell embryos and subsequent cultivation.

4. Process according to one of the preceding claims, **characterised in that** the embryonic stem cells are introduced into tetraploid blastocysts by microinjection.

5. Process according to one of claims 1 to 4, **characterised in that** genetically manipulated embryonic stem cells are used.

6. Process according to claim 5, **characterised in that** the genetically manipulated embryonic stem cells have been obtained by the introduction of plasmids which carry the desired genetic change.

## Revendications

1. Procédé pour l'obtention de mammifères non - humains, qui sont dérivés de cellules souches embryonnaires, avec des propriétés génétiques définies, en particulier des mammifères transgéniques, dans lequel des cellules souches embryonnaires de la même espèce de mammifère sont introduites dans les blastocystes et l'embryon obtenu est transféré dans une mère- nourricière,
**caractérisé en ce que** des cellules souches embryonnaires ayant des propriétés génétiques définies sont introduites dans des blastocystes tétraploïdes.

2. Procédé selon la revendication 1, **caractérisé en ce que** les mammifères sont des souris.

3. Procédé selon la revendication 1 ou 2, **caractérisé en ce que** l'on utilise des blastocystes qui ont été obtenus au moyen de l'électrofusion d'embryons bicellulaires et ont consécutivement été mis en culture.

4. Procédé selon l'une des revendications précédentes, **caractérisé en ce que** les cellules souches embryonnaires sont introduites par micro - injection dans les blastocystes tétraploïdes.

5. Procédé selon l'une des revendications 1 à 4, **caractérisé en ce que** l'on utilise des cellules souches embryonnaires génétiquement manipulées.

6. Procédé selon la revendication 5, **caractérisé en ce que** les cellules souches embryonnaires génétiquement manipulées ont été obtenues par introduction de plasmides, qui portent la modification génétique souhaitée.
